# EUROPÄISCHE PATENTSCHRIFT

(11) **EP 3 363 395 B1**
(45) Veröffentlichungstag und Bekanntmachung des Hinweises auf die Patenterteilung: **19.11.2025**
(21) Anmeldenummer: 18152870.4
(22) Anmeldetag: 23.01.2018
(51) Int. Cl.: A61B 18/14, A61B 18/00, A61B 17/32

(54) **SONDENEINRICHTUNG FÜR EIN RESEKTOSKOP ODER EIN ANDERES MIKROINVASIVES INSTRUMENT**
PROBE DEVICE FOR RESECTOSCOPE OR OTHER MICRO-INVASIVE INSTRUMENT
DISPOSITIF SONDE POUR UN RÉSECTOSCOPE OU POUR TOUT AUTRE INSTRUMENT MICROINVASIF

(30) Priorität: 21.02.2017 DE 102017103545
(43) Veröffentlichungstag der Anmeldung: 22.08.2018
(73) Patentinhaber: Karl Storz SE & Co. KG, 78532 Tuttlingen (DE)
(72) Erfinder: Rehbein, Stefan, 78532 Tuttlingen (DE)

(56) Entgegenhaltungen:
- CN-A- 105 250 023
- DE-A1- 102011 079 867
- GB-A- 2 335 858

## Beschreibung

Die vorliegende Erfindung ist auf eine Sondeneinrichtung für ein Resektoskop oder ein anderes mikroinvasives medizinisches Instrument zur Bearbeitung oder Manipulation von Gewebe und auf ein mikroinvasives Instrument mit einer solchen Sondeneinrichtung bezogen.

Ein Resektoskop für die transurethrale Resektion oder für gynäkologische oder andere Anwendungen umfasst einen Resektoskopschaft, der beispielsweise in die Harnröhre eingeführt werden kann. In dem Resektoskopschaft sind der Schaft eines Endoskops und der Schaft einer Sondeneinrichtung angeordnet. Mittels einer als Arbeitselement bezeichneten Einrichtung am proximalen Ende des Resektoskopschafts kann der Schaft der Sondeneinrichtung in dem Resektoskopschaft bewegt werden. Am distalen Ende der Sondeneinrichtung und damit auch im Bereich des distalen Endes des Resektoskopschafts ist eine Wirkeinrichtung vorgesehen, die mittels des Arbeitselements relativ zu dem distalen Ende des Resektoskopschafts bewegt werden kann. Die Wirkeinrichtung umfasst beispielsweise eine oder mehrere Elektroden für die monopolare bzw. bipolare Hochfrequenz-Chirurgie und die Elektrokauterisierung von durchtrenntem Gewebe. Alternativ kann die Wirkeinrichtung beispielsweise eine Lichtaustrittsfläche, durch die das intensive Licht eines Lasers austreten kann, aufweisen.

Ein derartiges Resektoskop ist in der Regel weitgehend zerlegbar, um eine vollständige Reinigung zu vereinfachen, eine defekte Komponente austauschen oder das Resektoskop an die Gewohnheiten des medizinischen Personals oder die spezifischen Erfordernisse einer medizinischen Maßnahme anpassen zu können. Insbesondere können in der Regel das proximale Ende des Resektoskopschafts von dem distalen Ende des Arbeitselements getrennt und das Endoskop und die Sondeneinrichtung aus dem Resektoskopschaft entnommen, von den übrigen Komponenten getrennt und ausgetauscht werden. Auch während eines medizinischen Eingriffs kann das proximale Ende des Resektoskopschafts von dem distalen Ende des Arbeitselementes vorübergehend getrennt werden, beispielsweise um einen Teil des bei der medizinischen Maßnahme verwendeten Spülfluids abzulassen.

Trotz der weitgehenden Zerlegbarkeit und der Bewegbarkeit des Schafts der Sonde in dem Resektoskopschaft muss das proximale Ende des Resektoskopschafts fluiddicht verschlossen sein. Dazu dient bei einem herkömmlichen Resektoskop eine Dichteinrichtung aus einem elastischen Material. Die Dichteinrichtung umfasst einen Kanal für den Schaft der Sondeneinrichtung und einen von ihrem Umfang bis zu dem Kanal reichenden Schlitz, durch den der Schaft der Sondeneinrichtung von der Seite her in den Kanal eingeführt werden kann. Wenn das proximale Ende des Resektoskopschafts mit dem distalen Ende des Arbeitselements verbunden wird, wird die Dichteinrichtung in axialer Richtung komprimiert, so dass der Schlitz geschlossen wird und die Dichteinrichtung an dem Schaft der Sondeneinrichtung sowie an Dichtflächen am proximalen Ende des Resektoskopschafts und an dem distalen Ende des Arbeitselements anliegt.

Ein Nachteil dieser Anordnung besteht darin, dass zur Kompression der Dichteinrichtung beim Verbinden des proximalen Endes des Resektoskopschafts mit dem distalen Ende des Arbeitselements Kraft erforderlich ist. Durch resultierende unwillkürliche Bewegungen des Resektoskopschafts kann der Patient verletzt werden. Ein weiterer Nachteil besteht darin, dass Reibung zwischen der elastischen Dichteinrichtung und dem Schaft der Sondeneinrichtung einer Bewegung des Schafts der Sondeneinrichtung relativ zu der Dichteinrichtung und damit relativ zu dem Resektoskopschaft einen Widerstand entgegensetzt, der eine feinfühlige Bewegung der Sondeneinrichtung erschwert.

CN105250023A zeigt eine Sondeneinrichtung für ein Resektoskop mit einem Instrumentenschaft und einem Sondenschaft darin, wobei dem distalen Instrumentenschaftende das distale Sondenschaftende mit einer Wirkeinrichtung benachbart ist. Ferner umfasst das Resektoskop eine Dichteinrichtung mit einem Kanal, in dem der Sondenschaft angeordnet ist, wobei der Sondenschaft relativ zu der Dichteinrichtung entlang der Sondenschaftachse verschiebbar ist und die Dichteinrichtung mit dem Sondenschaft verbunden ist.

Eine Aufgabe der vorliegenden Erfindung besteht darin, eine verbesserte Sondeneinrichtung für ein Resektoskop oder für ein anderes mikroinvasives medizinisches Instrument zur Bearbeitung oder Manipulation von Gewebe und ein verbessertes mikroinvasives medizinisches Instrument zu schaffen.

Diese Aufgabe wird durch die Gegenstände der unabhängigen Ansprüche gelöst.

Weiterbildungen sind in den abhängigen Ansprüchen angegeben.

Herkömmlich beruht die zuverlässige Dichtwirkung der Dichteinrichtung auf deren Elastizität. Nur diese Elastizität ermöglicht auch ein Einführen des Sondenschafts in den dafür vorgesehenen Kanal in der Dichteinrichtung durch einen Schlitz von der Seite her (d. h. in radialer Richtung). Ausführungsformen der vorliegenden Erfindung beruhen auf der Idee, anstelle einer elastischen Dichteinrichtung eine Dichteinrichtung aus einem Metall oder einer Keramik oder aus einem anderen nicht elastischen Material vorzusehen und dabei abweichend von einem herkömmlichen Resektoskop hinzunehmen, dass der Sondenschaft nicht mehr in radialer Richtung durch einen Schlitz hindurch aus dem Sondenschaftkanal in der Dichteinrichtung entnommen werden kann.

Eine Sondeneinrichtung für ein Resektoskop oder ein anderes mikroinvasives medizinisches Instrument zur Bearbeitung oder Manipulation von Gewebe umfasst einen Sondenschaft mit einem zylindrischen Abschnitt zur Anordnung in einem Instrumentenschaft und mit einem distalen Ende zur Anordnung nahe einem distalen Ende des Instrumentenschafts, eine Wirkeinrichtung an dem distalen Ende des Sondenschafts und eine Dichteinrichtung mit einem Sondenschaftkanal, in dem der zylindrische Abschnitt des Sondenschafts angeordnet ist, wobei der Sondenschaft relativ zu der Dichteinrichtung parallel zu der Längsachse des Sondenschafts und parallel zu der Längsachse des Sondenschaftkanals verschiebbar ist, wobei die Dichteinrichtung aus Metall oder Keramik oder aus einem anderen nicht elastischen Material gebildet und dauerhaft mit dem Sondenschaft verbunden ist.

Die Sondeneinrichtung ist insbesondere für ein Resektoskop zur transurethralen Resektion von erkranktem Gewebe aus Harnblase oder Prostata oder für gynäkologische oder andere Anwendungen vorgesehen und ausgebildet. Der zylindrische und damit auch gerade Abschnitt des Sondenschafts ist insbesondere starr ausgebildet, um nicht nur Zug-, sondern auch Schubkräfte aufnehmen und zu der Wirkeinrichtung übertragen zu können. Die Querschnittsfläche des zylindrischen Abschnitts des Sondenschafts und die Querschnittsfläche des Sondenschaftkanals der Dichteinrichtung entsprechen einander weitgehend. Die Querschnittsfläche des zylindrischen Abschnitts des Sondenschafts ist insbesondere nur wenig kleiner als die Querschnittsfläche des Sondenschaftkanals, um eine spiel- und reibungsarme Führung des Sondenschafts in dem Sondenschaftkanal zu bewirken. Die Querschnittsfläche des zylindrischen Abschnitts des Sondenschafts und die korrespondierende Querschnittsfläche des Sondenschaftkanals sind insbesondere jeweils kreisförmig. Alternativ können die Querschnittsfläche des zylindrischen Abschnitts des Sondenschafts und die korrespondierende Querschnittsfläche des Sondenschaftkanals jeweils nichtkreisförmig sein, beispielsweise polygonal oder elliptisch.

Die Wirkeinrichtung kann eine Hochfrequenzelektrode für die elektrochirurgische Durchtrennung von Gewebe und/oder zur Elektrokauterisation von durchtrenntem Gewebe und/oder eine Lasersonde mit einer Lichtaustrittsfläche für Laserlicht umfassen. Die Sondeneinrichtung weist insbesondere an der Wirkeinrichtung eine größere Querschnittsfläche auf als an dem zylindrischen Abschnitt des Sondenschafts. Insbesondere kann eine Hochfrequenz-Elektrode (beispielsweise in Gestalt einer Schleife) eine Breite aufweisen, die wesentlich größer ist als jede lineare Abmessung der Querschnittsfläche des zylindrischen Abschnitts des Sondenschafts.

Die Sondeneinrichtung kann ferner eine Klammer oder eine andere Führungseinrichtung zur formschlüssigen Führung der Sondeneinrichtung an dem Schaft eines Endoskops oder an einem Rohr, in dem der Schaft eines Endoskops angeordnet sein kann, oder an einer anderen als Führungsschiene wirkenden Einrichtung in einem Instrumentenschaft aufweisen. Diese Klammer oder andere Führungseinrichtung ist insbesondere nahe dem distalen Ende der Sondeneinrichtung zwischen der Wirkeinrichtung und dem zylindrischen Abschnitt des Sondenschafts angeordnet. Im Bereich der Klammer oder anderen Führungseinrichtung weist die Sondeneinrichtung einen größeren Querschnitt auf als im zylindrischen Abschnitt des Sondenschafts.

Die Sondeneinrichtung kann ferner proximal des zylindrischen Abschnitts einen flexiblen Leitungsabschnitt zum Übertragen von elektrischer oder optischer Leistung aufweisen. An dem proximalen Ende des flexiblen Leitungsabschnitts kann ein Steckverbinder zur elektrischen und/oder optischen Kopplung mit einer Leistungsquelle vorgesehen sein.

Das nicht elastische Material der Dichteinrichtung ist insbesondere insofern nicht elastisch, als die Dichteinrichtung bei der vorgesehenen Verwendung nicht oder nicht wesentlich verformt wird. Das elastische Material der Dichteinrichtung weist insbesondere einen Elastizitätsmodul (E-Modul), der größer als 5 GPa oder größer als 10 GPa oder größer als 20 GPa oder größer als 50 GPa ist, oder einen Schubmodul (G-Modul), der größer als 1 GPa oder größer als 5 GPa oder größer als 10 GPa oder größer als 20 GPa ist, auf. Die Dichteinrichtung ist insbesondere nicht aus einem Gummi oder einem Silikonkautschuk oder -elastomer oder einem anderen Elastomer gebildet.

Die Dichteinrichtung aus dem nicht elastischen Material wird beim Verbinden des proximalen Endes des Instrumentenschafts mit dem distalen Ende des Arbeitselements nicht oder nicht wesentlich verformt.

Die innere Oberfläche des Sondenschaftkanals der Dichteinrichtung liegt nicht vollflächig an der äußeren Oberfläche des zylindrischen Abschnitts des Sondenschafts an. Vielmehr verbleibt ein kleiner ringförmiger Spalt zwischen der äußeren Oberfläche des zylindrischen Abschnitts des Sondenschafts und der inneren Oberfläche des Sondenschaftkanals der Dichteinrichtung. Aufgrund des nicht elastischen Materials der Dichteinrichtung und ihrer resultierenden Formstabilität kann dieser ringförmige Spalt jedoch so schmal gewählt werden, dass einerseits eine reibungsarme Verschiebung des zylindrischen Abschnitts des Sondenschafts in dem Sondenschaftkanal der Dichteinrichtung möglich ist und andererseits nur eine sehr kleine Menge einer Spülflüssigkeit oder eines anderen Fluids durch den ringförmigen Kanal hindurchtreten kann.

Der Sondenschaft und die Dichteinrichtung der Sondeneinrichtung sind insofern dauerhaft miteinander verbunden, als sie nicht zerstörungsfrei von einander trennbar sind. Der Sondenschaft kann insbesondere nicht vollständig aus dem Sondenschaftkanal der Dichteinrichtung herausgezogen werden, beispielsweise weil er sowohl distal als auch proximal seines zylindrischen Abschnitts jeweils einen Querschnitt aufweist, der größer als der Querschnitt des Sondenschaftkanals ist. Distal kann dieser größere Querschnitt beispielsweise bei der Wirkeinrichtung und/oder bei der erwähnten Klammer oder anderen Führungseinrichtung vorliegen. Proximal kann dieser größere Querschnitt beispielsweise bei einem Steckverbinder zur lösbaren mechanischen und elektrischen und/oder optischen Verbindung mit einer Leistungsquelle vorliegen.

Da die Dichtwirkung der Dichteinrichtung aus dem nicht elastischen Material nicht auf einer Kompression und Verformung der Dichteinrichtung beruht, können die Sondeneinrichtung, das proximale Ende des Instrumentenschafts und das Arbeitselement so ausgebildet sein, dass beim Verbinden des proximalen Endes des Endoskopschafts mit dem distalen Ende des Arbeitselements keine oder nur eine - insbesondere im Vergleich zu einer herkömmlichen Dichteinrichtung aus einem elastischen Material - kleine Kraft erforderlich ist. Eine unwillkürliche Bewegung des Instrumentenschafts beim Öffnen und Schließen der Verbindung zu dem Arbeitselement und damit eine mögliche Verletzung eines Patienten werden somit deutlich unwahrscheinlicher.

Bei einer Sondeneinrichtung, wie sie hier beschrieben ist, ist die Dichteinrichtung insbesondere nicht geschlitzt.

Aufgrund des nicht elastischen Materials der Dichteinrichtung könnte ein Schlitz nicht durch Kompression und elastische Verformung der Dichteinrichtung geschlossen werden. Ein Einlegen des zylindrischen Abschnitts des Sondenschafts in den Sondenschaftkanal in der Dichteinrichtung von der Seite her ist deshalb nicht möglich. Stattdessen bleibt die Dichteinrichtung dauerhaft mit dem Sondenschaft verbunden.

Bei einer Sondeneinrichtung, wie sie hier beschrieben ist, weist die Dichteinrichtung insbesondere ferner einen Endoskopschaftkanal zum Aufnehmen eines Endoskopschafts oder zum Aufnehmen eines Rohrs, in dem ein Endoskopschaft angeordnet sein kann, auf.

Der Querschnitt des Endoskopschaftkanals ist so an den Querschnitt des Rohrs zum Aufnehmen eines Endoskopschafts oder an den Querschnitt eines Endoskops, für den das mikroinvasive Instrument und damit die Sondeneinrichtung vorgesehen sind, angepasst, dass der Endoskopschaft oder das Rohr zur Aufnahme eines Endoskopschafts spiel- und reibungsarm in dem Endoskopschaftkanal der Dichteinrichtung geführt ist und durch einen verbleibenden ringförmigen Kanal nur wenig Flüssigkeit oder anderes Fluid hindurchtreten kann.

Bei einer Sondeneinrichtung, wie sie hier beschrieben ist, weist der Endoskopschaftkanal insbesondere eine ringförmige Erweiterung auf, in der ein Dichtelement angeordnet ist.

Das Dichtelement ist insbesondere als O-Ring aus Teflon, Nitrilkautschuk, Silikonkautschuk, Silikonelastomer oder einem anderen Elastomer oder aus einem anderen elastischen Material ausgebildet. Das Dichtelement weist insbesondere eine toroidale Gestalt oder eine andere ringförmige Gestalt mit kreisförmigem, rundem oder eckigem Querschnitt auf.

Bei einer Sondeneinrichtung, wie sie hier beschrieben ist, weist die Dichteinrichtung insbesondere eine konische Dichtfläche auf, die zur dichtenden Anlage an einer korrespondierenden konischen Dichtfläche an einem Instrumentenschaft vorgesehen und ausgebildet ist.

Der Durchmesser der konischen Dichtfläche nimmt insbesondere nach distal ab. Zahlreiche herkömmliche Instrumentenschäfte weisen an ihren proximalen Enden eine konische Dichtfläche auf. Die Sondeneinrichtung kann mit einem solchen herkömmlichen Instrumentenschaft kombinierbar sein.

Bei einer Sondeneinrichtung, wie sie hier beschrieben ist, weist der Sondenschaftkanal insbesondere eine ringförmige Erweiterung auf, in der ein Dichtelement angeordnet ist.

Das Dichtelement ist insbesondere als O-Ring aus Teflon, Nitrilkautschuk, Silikonkautschuk, Silikonelastomer oder einem anderen Elastomer oder aus einem anderen elastischen Material ausgebildet. Das Dichtelement weist insbesondere die Gestalt eines Toroids oder eines anderen Rings mit einem kreisförmigen, runden oder eckigen Querschnitt auf. Das Dichtelement kann den ringförmigen Spalt zwischen der äußeren Oberfläche des zylindrischen Abschnitts des Sondenschafts und der inneren Oberfläche des Sondenschaftkanals der Dichteinrichtung schließen und so die Dichtwirkung noch weiter verbessern.

Da das Dichtelement in der Dichteinrichtung beim Verbinden des proximalen Endes eines Instrumentenschafts mit dem distalen Ende eines Arbeitselements nicht verformt wird, setzt es dieser Verbindung auch keinen Widerstand entgegen. Da das Dichtelement auch nicht an die äußere Oberfläche des zylindrischen Abschnitts des Sondenschafts gepresst wird, muss es die Reibung zwischen dem zylindrischen Abschnitt des Sondenschafts und der Dichteinrichtung auch nicht wesentlich erhöhen.

Bei einer Sondeneinrichtung, wie sie hier beschrieben ist, weist die Dichteinrichtung insbesondere eine Kupplungseinrichtung zur lösbaren mechanischen Verbindung der Dichteinrichtung mit dem distalen Ende eines Arbeitselements eines Resektoskops oder eines anderen mikroinvasiven Instruments auf.

Die durch die Kupplungseinrichtung bewirkte mechanische Verbindung zwischen der Dichteinrichtung und dem Arbeitselement kann verhindern, dass bei einem Trennen des proximalen Endes des Instrumentenschafts von dem distalen Ende des Arbeitselements die Dichteinrichtung an dem proximalen Ende des Instrumentenschafts verbleibt. Die durch die Kupplungseinrichtung bewirkte mechanische Verbindung zwischen der Dichteinrichtung und dem Arbeitselement kann gewährleisten, dass die Dichteinrichtung stattdessen an dem distalen Ende des Arbeitselements verbleibt, von dem sie anschließend manuell getrennt werden kann.

Bei einer Sondeneinrichtung, wie sie hier beschrieben ist, umfasst die Dichteinrichtung insbesondere eine elastische Rasteinrichtung zur Ausbildung einer Rastverbindung mit dem distalen Ende des Arbeitselements des Resektoskops oder des anderen mikroinvasiven Instruments.

Bei einer Sondeneinrichtung, wie sie hier beschrieben ist, weist die Kupplungseinrichtung insbesondere eine Nut oder eine andere Ausnehmung zur Aufnahme eines elastischen Rastelements an dem distalen Ende eines Arbeitselements eines Resektoskops oder des anderen mikroinvasiven Instruments auf.

Die Rasteinrichtung umfasst beispielsweise eine Rastnase an einem Ende eines biegeelastischen Holms, die in eine Nut oder eine andere Ausnehmung an dem distalen Ende des Arbeitselements eingreifen kann. Die Rastwirkung kann über die elastischen Eigenschaften des elastischen Holms oder der anderen elastischen Rasteinrichtung so eingestellt werden, dass die Dichteinrichtung einerseits beim Trennen des Instrumentenschafts von dem Arbeitselement sicher an dem Arbeitselement verbleibt, andererseits aber anschließend leicht manuell von dem Arbeitselement getrennt werden kann.

Bei einer Sondeneinrichtung, wie sie hier beschrieben ist, umfasst die Dichteinrichtung insbesondere eine Knagge oder eine L-förmige Nut zur Ausbildung einer Renkverbindung mit dem distalen Ende des Arbeitselements des Resektoskops oder des anderen mikroinvasiven Instruments.

Um das Herstellen und Lösen einer Renkverbindung zwischen der Dichteinrichtung und dem distalen Ende des Arbeitselements des mikroinvasiven Instruments zu ermöglichen, ist das distale Ende des Arbeitselements insbesondere so ausgebildet, dass der Sondenschaft und ggf. ein Endoskopschaft, die jeweils spielarm in der Dichteinrichtung geführt sind, relativ zu dem distalen Ende des Arbeitselements entlang bogenförmiger Bahnen bewegt werden können.

Ein mikroinvasives medizinisches Instrument zur Bearbeitung oder Manipulation von Gewebe umfasst einen Instrumentenschaft mit einem distalen Ende und einem proximalen Ende, ein Arbeitselement zur lösbaren Verbindung mit dem proximalen Ende des Instrumentenschafts und zur lösbaren Verbindung mit einem proximalen Ende eines Endoskops und zur lösbaren Verbindung mit einem proximalen Ende eines Sondenschafts und zur manuellen Bewegung des Sondenschafts relativ zu dem Instrumentenschaft, wobei zwischen dem distalen Ende des Arbeitselements und dem proximalen Ende des Instrumentenschafts ein Raum zu Aufnahme der Dichteinrichtung einer Sondeneinrichtung nach einem der vorangehenden Ansprüche vorgesehen ist.

Das distale Ende des Arbeitselements weist dazu insbesondere Merkmale auf, die zu beschriebenen Merkmalen der Sondeneinrichtung, insbesondere der Dichteinrichtung, korrespondieren.

Bei einem mikroinvasiven Instrument, wie es hier beschrieben ist, ist an dem proximalen Ende des Instrumentenschafts insbesondere eine konische Dichtfläche vorgesehen, die zumindest teilweise zu einer konischen Dichtfläche einer Dichteinrichtung einer Sondeneinrichtung, wie sie hier beschrieben ist, korrespondiert.

Die konische Dichtfläche an dem proximalen Ende des Instrumentenschafts korrespondiert insbesondere nicht oder nur teilweise zu einer konischen Fläche an dem Arbeitselement. Ein Teil der konischen Dichtfläche an dem proximalen Ende des Instrumentenschafts oder die gesamte konische Dichtfläche an dem proximalen Ende des Instrumentenschafts korrespondiert zu einer konischen Dichtfläche einer Dichteinrichtung einer Sondeneinrichtung, wie sie hier beschrieben ist.

Bei einem mikroinvasiven Instrument, wie es hier beschrieben ist, ist insbesondere an dem distalen Ende des Arbeitselements eine Öffnung für den Sondenschaft oder für den Endoskopschaft vorgesehen, wobei die Öffnung für den Sondenschaft oder für den Endoskopschaft einen Querschnitt aufweist, der eine Bewegung des Sondenschafts oder des Endoskopschafts in der Öffnung bei einem Rotieren der Dichteinrichtung relativ zu dem distalen Ende des Arbeitselements ermöglicht.

Die Öffnung weist insbesondere einen Querschnitt auf, der durch Verschiebung des Querschnitts des Sondenschafts oder des Endoskopschafts entlang eines Kreisbogenabschnitts, dessen Mittelpunkt auf der Achse, um die die Dichteinrichtung beim Herstellen oder Lösen einer Renkverbindung mit dem distalen Ende des Arbeitselements zu rotieren ist, entsteht.

Das mikroinvasive Instrument ist insbesondere ein Resektoskop.

### Kurzbeschreibung der Figuren

Nachfolgend werden Ausführungsformen anhand der beigefügten Figuren näher erläutert. Es zeigen:
- Figur 1: eine schematische Darstellung eines Längsschnitts durch einen Teil eines Resektoskops;
- Figur 2: eine schematische Darstellung eines Längsschnitts durch das Resektoskop aus Figur 1 in einer anderen Konfiguration;
- Figur 3: eine schematische Darstellung eines Querschnitts durch das Resektoskop aus den Figuren 1 und 2;
- Figur 4: eine schematische Darstellung eines Längsschnitts durch einen Teil eines Resektoskops;
- Figur 5: eine schematische Darstellung eines Längsschnitts durch das Resektoskop aus Figur 4 in anderen Konfiguration;
- Figur 6: eine schematische Darstellung eines Querschnitts durch das Resektoskop aus den Figuren 4 und 5;
- Figur 7: eine schematische Darstellung eines Längsschnitts durch einen Teil eines weiteren Resektoskops;
- Figur 8: eine schematische Darstellung eines Längsschnitts durch das Resektoskop aus Figur 7 in einer anderen Konfiguration;
- Figur 9: eine schematische Darstellung eines Querschnitts durch das Resektoskop aus den Figuren 7 und 8;
- Figur 10: eine schematische Darstellung eines Längsschnitts durch einen Teil eines weiteren Resektoskops;
- Figur 11: eine schematische Darstellung eines Längsschnitts durch das Resektoskop aus Figur 10 in einer weiteren Konfiguration;
- Figur 12: eine schematische Darstellung eines Querschnitts durch das Resektoskop aus den Figuren 10 und 11.

### Beschreibung der Ausführungsformen

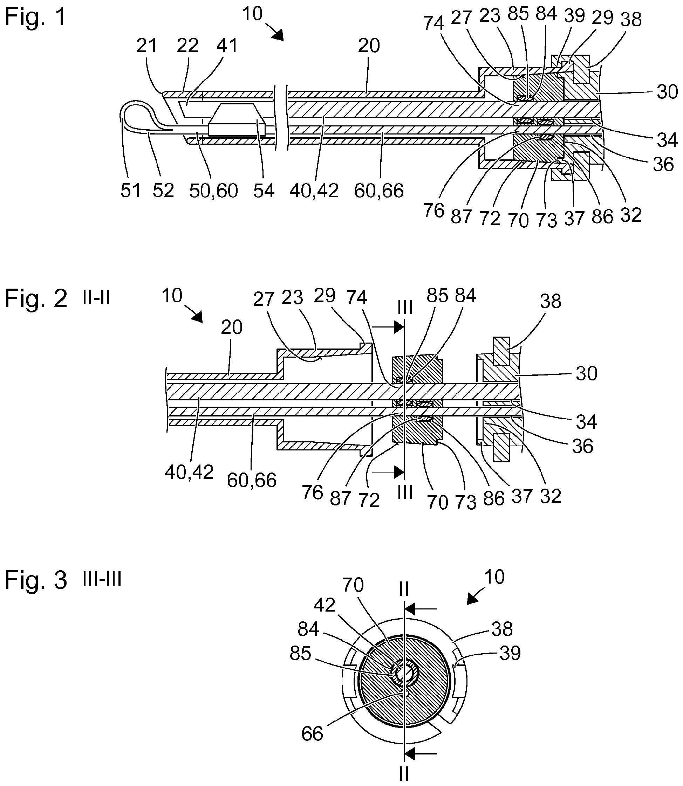

Figur 1 zeigt eine schematische Darstellung eines Längsschnitts durch einen mittleren Bereich und durch das distale (in Figur 1 links dargestellte) Ende eines Resektoskops 10 als Beispiel eines mikroinvasiven Instruments, bei dem eine Wirkeinrichtung gegenüber einem Schaft verschiebbar ist, um Gewebe zu bearbeiten oder zu manipulieren. Das distale Ende des Resektoskops 10 ist nur teilweise im Schnitt dargestellt.

Das Resektoskop 10 umfasst einen im Wesentlichen rohrförmigen Resektoskopschaft 20 mit einem distalen Ende 21 und einem proximalen Ende 23. Das distale Ende 21 des Resektoskopschafts 20 wird durch einen Abschnitt 22 aus einem elektrisch isolierenden Material gebildet. Der Resektoskopschaft 20 ist an seinem proximalen Ende 23 becherförmig erweitert und weist an seiner Innenseite eine konische Dichtfläche 27 auf. An dem äußerst proximalen Rand des Resektoskopschafts 20 sind zwei oder mehr Knaggen 29 vorgesehen, die nach radial außen überstehen.

Das Resektoskop 10 umfasst ferner ein Arbeitselement 30, von dem in Figur 1 nur das distale Ende 32 sichtbar ist, das mit dem proximalen Ende 23 des Resektoskopschafts 20 mechanisch verbunden ist.

Ferner umfasst das Resektoskop 10 ein Endoskop 40, von dem in Figur 1 nur ein Endoskopschaft 42 sichtbar ist. Der Endoskopschaft 42 ist überwiegend innerhalb des Resektoskopschafts 20 angeordnet. Das distale Ende 41 des Endoskops 40 ist in oder nahe dem distalen Ende 21 des Resektoskopschafts 20 angeordnet. Der distale Bereich des Endoskopschafts 42 ist im Gegensatz zu dem übrigen Bereich des Endoskopschafts 42 und zu anderen Komponenten des Resektoskops 10 nicht im Schnitt dargestellt.

Ferner umfasst das Resektoskop 10 eine Sondeneinrichtung 50, von der in Figur 1 nur ein distales Ende 51 und - teilweise - ein Sondenschaft 60 sichtbar ist. Der Sondenschaft 60 ist überwiegend innerhalb des Resektoskopschafts 20 angeordnet. Der Sondenschaft 60 umfasst einen zylindrischer Abschnitt 66, der sich über einen großen Teil der Länge des Resektoskopschafts 20 erstreckt. Der distale Bereich des Sondenschafts 60 ist im Gegensatz zu dem zylindrischer Abschnitt 66 des Sondenschafts 60 nicht im Schnitt dargestellt.

Das distale Ende 51 der Sondeneinrichtung 50 wird bei dem dargestellten Beispiel durch eine schleifen- oder schlaufenförmige Elektrode 52 für elektrochirurgische Anwendungen als Wirkeinrichtung gebildet. Die Elektrode 52 weist Abmessungen auf, die über den Querschnitt des zylindrischen Abschnitts 66 des Sondenschafts 60 deutlich hinausgehen. Alternativ oder zusätzlich kann an dem distalen Ende 51 der Sondeneinrichtung als Wirkeinrichtung eine Lichtaustrittsfläche eines Lichtleiters, durch die intensives Laserlicht zum Schneiden, Abtragen oder Veröden von Gewebe austreten kann, vorgesehen sein.

Ferner weist die Sondeneinrichtung 50 bei dem dargestellten Beispiel eine Führungseinrichtung 54 auf. Die Führungseinrichtung 54 weist die Gestalt eine Klammer auf, die mit dem Sondenschaft 60 starr verbunden ist und den Endoskopschaft 42 zu mehr als der Hälfte umgreift. Die Führungseinrichtung 54 ist an dem Endoskopschaft 42 formschlüssig geführt, so dass die Sondeneinrichtung 50 zwar relativ zu dem Endoskopschaft 42 parallel zu der Längsachse des Endoskopschafts 42, aber nicht oder nur eingeschränkt in andere Richtungen bewegt werden kann. Abweichend von der Darstellung in Figur 1 kann die Führungseinrichtung 54 den Endoskopschaft 42 vollständig umgreifen. Auch die Abmessungen der Führungseinrichtung 54 gehen über den Querschnitt des zylindrischen Abschnitts 66 des Sondenschafts 60 deutlich hinaus.

Der Endoskopschaft 42 und der Sondenschaft 60 erstrecken sich im Wesentlichen über die gesamte Länge des Resektoskopschafts 20. Der Querschnitt des Resektoskopschafts 20 ist so ausgebildet, dass der Resektoskopschaft 20 den Endoskopschaft 42 und den Sondenschaft 60 aufnehmen kann und ferner eine Spülflüssigkeit oder ein anderes Fluid durch den Resektoskopschaft 20 geleitet werden kann.

In dem distalen Ende 32 des Arbeitselements 30 sind eine erste Öffnung 34 in Gestalt eines zylindrischen Durchgangskanals für den Endoskopschaft 42 und eine zweite Öffnung 36 in Gestalt eines zylindrischen Durchgangskanals für den zylindrischen Abschnitt 66 des Sondenschafts 60 der Sondeneinrichtung 50 vorgesehen. Beide Öffnungen 34, 36 verlaufen parallel zueinander und parallel zu der Längsachse des Resektoskopschafts 20. Ferner ist an dem distalen Ende 32 des Arbeitselements 30 ein ringförmiger Kragen 37.

An dem distalen Ende 32 des Arbeitselements 30 ist ferner ein Kupplungsring 38 mit zwei oder mehr Knaggen 39 vorgesehen. Der Kupplungsring 38 ist um eine Rotationsachse in der Zeichenebene der Figur 1 rotierbar. Die Knaggen 39 an dem Kupplungsring 38 korrespondieren zu den Knaggen 29 an dem proximalen Ende 23 des Resektoskopschafts 20. Bei der in Figur 1 gezeigten Konfiguration hintergreifen die Knaggen 39 an dem Kupplungsring 38 die Knaggen 29 an dem proximalen Ende 23 des Resektoskopschafts 20, so dass das proximale Ende 23 des Resektoskopschafts 20 an dem distalen Ende 32 des Arbeitselements 30 formschlüssig gehalten ist. Diese Verbindung kann durch Rotation des Kupplungsrings 38 gelöst werden. Somit liegt eine durch die Knaggen 29 am proximalen Ende 23 des Resektoskopschafts 20 und die Knaggen 39 am Kupplungsring 38 gebildete Renkverbindung zwischen dem proximalen Ende 23 des Resektoskopschafts 20 und dem distalen Ende 32 des Arbeitselements 30 vor.

Die Dichteinrichtung 70 weist eine konische Dichtfläche 72 auf, deren Durchmesser nach distal (in Figur 1: links) abnimmt, und die zu der konischen Dichtfläche 27 an dem proximalen Ende 23 des Resektoskopschafts 20 korrespondiert. Ferner weist die Dichteinrichtung eine ringförmige Stufe 73 auf, die zu dem ringförmigen Kragen 37 an dem distalen Ende 32 des Arbeitselements 30 korrespondiert. Der in Figur 1 gezeigte Eingriff des Kragens 37 an dem distalen Ende 32 des Arbeitselements in die Stufe 73 an der Dichteinrichtung 70 bewirkt formschlüssig eine korrekte Positionierung der Dichteinrichtung 70 relativ zu dem distalen Ende 32 des Arbeitselements 30.

Ferner umfasst das Resektoskop 10 eine Dichteinrichtung 70. Die Dichteinrichtung 70 weist einen Endoskopschaftkanal 74 zur Aufnahme des Endoskopschafts 42 und einen Sondenschaftkanal 76 zur Aufnahme des zylindrischen Abschnitts 66 des Sondenschafts 60 auf. Der Querschnitt des Endoskopschaftkanals 74 ist so an dem Querschnitt des Endoskopschafts 42 angepasst, dass zwischen der inneren Oberfläche des Endoskopschaftkanals 74 und der äußeren Oberfläche des Endoskopschafts 42 nur ein kleiner ringförmiger Spalt verbleibt und der Endoskopschaft 42 spiel- und reibungsarm in dem Endoskopschaftkanal 74 geführt ist. Der Querschnitt des Sondenschaftkanals 76 ist so an den Querschnitt des zylindrischen Abschnitts 66 des Sondenschafts 60 angepasst, dass zwischen der inneren Oberfläche des Sondenschaftkanals 76 der Dichteinrichtung 70 und der äußeren Oberfläche des zylindrischen Abschnitts 66 des Sondenschafts 60 nur ein schmaler ringförmiger Spalt verbleibt und der zylindrische Abschnitt 66 des Sondenschafts 60 spiel- und reibungsarm in dem Sondenschaftkanal 76 geführt ist.

Die Dichteinrichtung 70 ist aus einem Metall, einer Keramik oder einem anderen nicht elastischen Material gebildet und wird bei der vorgesehenen Verwendung in dem Resektoskop 10 nicht oder nicht wesentlich verformt. Die geringe Elastizität der Dichteinrichtung ermöglicht eine präzise Einhaltung der vorgesehenen Geometrien des ringförmigen Spalts um den Endoskopschaft 42 in dem Endoskopschaftkanal 74 und des vorgesehenen Spalts um den zylindrischen Abschnitt 66 des Sondenschafts 60 in dem Sondenschaftkanal 76. Beide ringförmige Spalte können deshalb so schmal ausgebildet sein, dass nur wenig Fluid durch die ringförmigen Spalte hindurchtreten kann.

Bei dem in Figur 1 dargestellten Beispiel ist zusätzlich in dem Endoskopschaftkanal 74 eine ringförmige Nut 84, die den Querschnitt des Endoskopschaftkanals lokal vergrößert, vorgesehen. In der ringförmigen Nut 84 in dem Endoskopschaftkanal 74 ist ein O-Ring 85 aus Teflon, Nitrilkautschuk, Silikonkautschuk, Silikonelastomer oder einem anderen Elastomer oder aus einem anderen elastischen Material angeordnet. Der O-Ring 85 ist so dimensioniert und die elastischen Eigenschaften des O-Rings 85 sind so gewählt, dass er den ringförmigen Spalt zwischen der äußeren Oberfläche des Endoskopschafts 42 und der inneren Oberfläche des Endoskopschaftkanals 74 elastisch und fluiddicht verschließt.

Bei dem in Figur 1 dargestellten Beispiel ist ferner eine ringförmige Nut 86 in dem Sondenschaftkanal 76 vorgesehen, die den Querschnitt des Sondenschaftkanals 76 lokal vergrößert. In der ringförmigen Nut 86 in dem Sondenschaftkanal 76 ist ein O-Ring 87 aus Teflon, Nitrilkautschuk, Silikonkautschuk, Silikonelastomer oder einem anderen Elastomer oder einem anderen elastischen Material angeordnet. Der O-Ring 87 ist so dimensioniert und die elastischen Eigenschaften des O-Rings 87 sind so gewählt, dass der O-Ring 87 an der äußeren Oberfläche des zylindrischen Abschnitts 66 des Sondenschafts 60 anliegt und den ringförmigen Spalt zwischen der äußeren Oberfläche des zylindrischen Abschnitts 66 des Sondenschafts 60 und der inneren Oberfläche des Sondenschaftkanals 76 elastisch fluiddicht abdichtet.

Beim Zerlegen des Resektoskops 10 wird der Endoskopschaft 42 nach proximal (in Figur 1: rechts) aus dem Resektoskopschaft 20, der Dichteinrichtung 70 und dem Arbeitselement 30 herausgezogen. Dies ist möglich, weil der Endoskopschaft 42 eine durchgehend zylindrische Gestalt hat. In Gegensatz zu dem Endoskopschaft 42 weist die Sondeneinrichtung 50 an oder nahe ihrem distalen Ende 51 distalen Ende aufgrund einer Führungseinrichtung 54 und/oder einer Wirkeinrichtung 52 einen gegenüber dem zylindrischen Abschnitt 66 des Sondenschafts 60 deutlich vergrößerten Querschnitt auf. Eine Entnahme des Sondenschafts 60 nach proximal ist deshalb ausgeschlossen. In Gegensatz zu einer herkömmlichen Dichteinrichtung aus einem elastischen Material verbleibt die Dichteinrichtung 70 an dem Sondenschaft 60 und bildet somit mit diesem zusammen eine nicht zerstörungsfrei trennbare Einheit.

Das Arbeitselement 30 weist nahe dessen distalem Ende 32 einen in Figur 1 nicht sichtbaren Schlitz auf, der sich von einem äußeren Umfang des Arbeitselements 30 bis zu der zweiten Öffnung 36 erstreckt und ein Einlegen des zylindrischen Abschnitts 66 des Sondenschafts 60 in das Arbeitselement 30 von der Seite her ermöglicht.

Figur 2 zeigt eine weitere schematische Darstellung eines Längsschnitts durch den mittleren Bereich des Resektoskops 10 aus Figur 1. Die Schnittebene II-II der Figur 2 entspricht der Schnittebene der Figur 1. Die Darstellung in Figur 2 unterscheidet sich von der Darstellung in Figur 1 dadurch, dass eine andere Konfiguration gezeigt ist. Die in Figur 2 gezeigte Konfiguration liegt beispielsweise beim Zusammensetzen oder Zerlegen des Resektoskops 10 vor.

Ausgehend von der in Figur 1 gezeigten Konfiguration entsteht die in Figur 2 gezeigte Konfiguration, indem der Kupplungsring 38 gedreht wird, so dass die Knaggen 39 an den Kupplungsring 38 von den Knaggen 29 an dem proximalen Ende 23 des Resektoskopschafts 20 entfernt werden und die Renkverbindung zwischen dem proximalen Ende 23 des Resektoskopschafts 20 und dem distalen Ende 32 des Arbeitselements 30 getrennt wird. Danach können der Resektoskopschaft 20 und die Dichteinrichtung 70 relativ zu dem Arbeitselement 30 nach distal verschoben werden, beispielsweise bis zu den in Figur 2 gezeigten Positionen.

Ausgehend von der in Figur 2 gezeigten Konfiguration können der Resektoskopschaft 20 nach distal abgezogen, der Endoskopschaft 42 nach proximal herausgezogen werden und dann der zylindrische Abschnitt 66 des Sondenschafts 60 in einer Bewegung beispielsweise orthogonale zu der Zeichenebene der Figur 2 durch einen in Figur 2 nicht sichtbaren Schlitz in dem Arbeitselement 30 aus dem Arbeitselement 30 entnommen werden. Dabei verbleibt wie erwähnt der zylindrische Abschnitt 66 des Sondenschafts 60 in dem Sondenschaftkanal 76 der Dichteinrichtung.

Figur 3 zeigt eine schematische Darstellung eines weiteren Schnitts durch das Resektoskop 10 aus den Figuren 1 und 2 in der in Figur 2 gezeigten Konfiguration. Die Schnittebene III-III der Figur 3 ist orthogonal zu der Schnittebene II-II der Figur 2. Die Position der Schnittebene II-II der Figur 2 ist in Figur 3 angedeutet. Die Schnittebene III-III der Figur 3 ist in Figur 2 angedeutet.

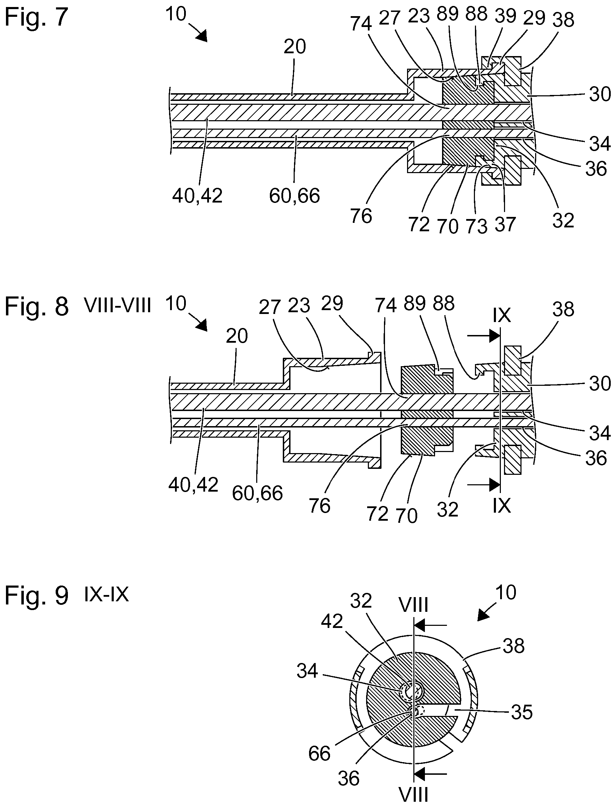

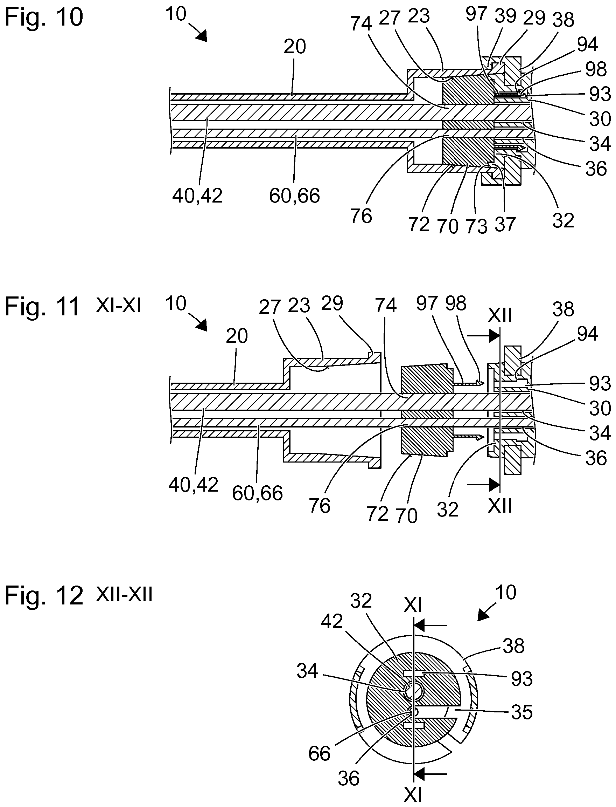

Die Schnittebene III-III der Figur 3 schneidet nur die Dichteinrichtung 70, und zwar im Bereich der ringförmigen Nut 84 in dem Endoskopschaftkanal 74 und des O-Rings 85 in der ringförmigen Nut 84. Die Querschnitte des Endoskopschafts 42 und des zylindrischen Abschnitts 66 des Sondenschafts 60 sind in Figur 3 vereinfachend als homogene schraffierte Flächen angedeutet. In Figur 3 sind ferner die Knaggen 39 an dem Kupplungsring 38 an dem distalen Ende 32 des Arbeitselements 30 (vgl. Figuren 1, 2) sichtbar. Der Kupplungsring weist einen Schlitz auf, dessen Funktion anhand der Figuren 9 und 12 deutlich wird.

Anders als herkömmliche Dichteinrichtungen für Resektoskope weist die Dichteinrichtung 70 keinen Schlitz auf, durch den der zylindrische Abschnitt 66 des Sondenschafts 60 von der Seite her in die Dichteinrichtung 70 eingesetzt werden könnte. Deshalb sind keine Kompression und keine elastische Verformung der Dichteinrichtung 70 erforderlich, um einen solchen Schlitz zuverlässig zu schließen. Die Dichteinrichtung 70 kann deshalb aus einem Metall, einer Keramik oder einem anderen nicht elastischen Material gebildet sein. Andererseits muss die Dichteinrichtung 70, wenn der Sondenschaft 60 an seinem distalen Ende - wie bei den Figuren 1 und 2 beschrieben - einen vergrößerten Querschnitt aufweist, an dem zylindrischen Abschnitt 66 des Sondenschafts 60 verbleiben. Der Sondenschaft 60 und die Dichteinrichtung 70 bilden deshalb eine nicht zerstörungsfrei trennbare Einheit.

Abweichend von der Darstellung in den Figuren 1 bis 3 kann die Dichteinrichtung nur entweder eine ringförmige Nut 84 in dem Endoskopschaftkanal 74 und einen O-Ring 85 in der ringförmigen Nut 84 oder eine ringförmige Nut 86 in dem Sondenschaftkanal 76 und einen O-Ring 87 in der ringförmigen Nut 86 aufweisen.

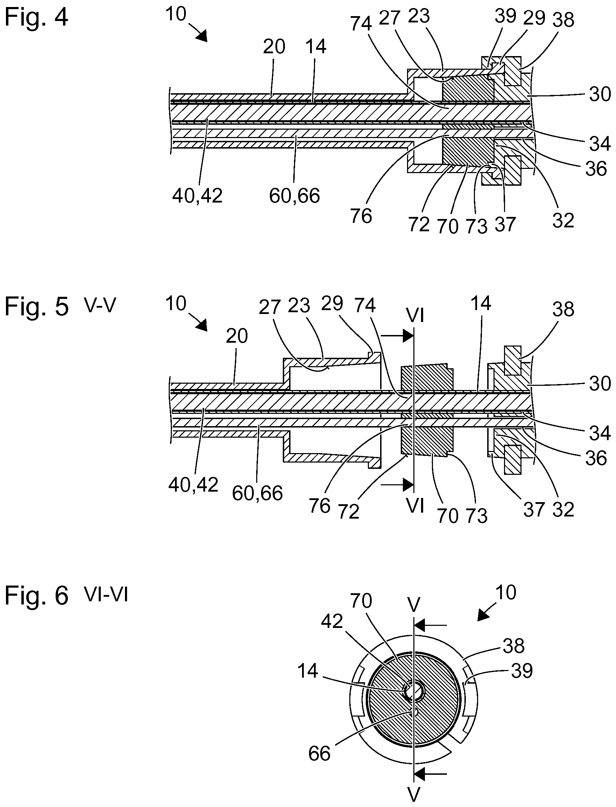

Figur 4 zeigt eine schematische Darstellung eines Längsschnitts durch einen mittleren Bereich eines weiteren Resektoskops 10, das in einigen Merkmalen, Eigenschaften und Funktionen dem anhand der Figuren 1 bis 3 dargestellten Resektoskop ähnelt, insbesondere auch hinsichtlich des in Figur 4 nicht dargestellten distalen Endes des Resektoskops 10.

Die Schnittebene der Figur 4 entspricht den Schnittebenen der Figuren 1 und 2. Die in Figur 4 dargestellte Konfiguration entspricht der in Figur 1 dargestellten Konfiguration. Nachfolgend sind insbesondere Merkmale, Eigenschaften und Funktionen des in Figur 4 gezeigten Resektoskops beschrieben, die sich von denjenigen des anhand der Figuren 1 bis 3 dargestellten Resektoskops unterscheiden.

Das in Figur 4 gezeigte Resektoskop 10 unterscheidet sich von dem anhand der Figuren 1 bis 3 dargestellten Resektoskop insbesondere dadurch, dass es ein Führungsrohr 14 für den Endoskopschaft 42 umfasst.

Das in Figur 4 gezeigte Resektoskop 10 unterscheidet sich von dem anhand der Figuren 1 bis 3 dargestellten Resektoskop ferner dadurch, dass weder in dem Endoskopschaftkanal 74 noch in dem Sondenschaftkanal 76 der Dichteinrichtung 70 ringförmige Nuten oder O-Ringe vorgesehen sind. Die Dichtwirkung der Dichteinrichtung 70 beruht somit allein auf den geringen Breiten der ringförmigen Spalte um den zylindrischen Abschnitt 66 des Sondenschafts 60 und um den Endoskopschaft 42 des Endoskops 40 oder um das Führungsrohr 14.

Figur 5 zeigt eine schematische Darstellung eines weiteren Schnitts durch das anhand der Figur 4 dargestellte Resektoskop 10. Die Schnittebene V-V der Figur 5 entspricht der Schnittebene der Figur 4. Die in Figur 5 gezeigte Konfiguration entspricht der in Figur 2 gezeigten Konfiguration.

Das Führungsrohr 14 kann entweder mit dem Resektoskopschaft 20 oder mit der Dichteinrichtung 70 oder mit dem Arbeitselement 30 starr und dauerhaft verbunden sein.

Figur 6 zeigt eine schematische Darstellung eines weiteren Schnitts durch das anhand der Figuren 4 und 5 dargestellte Resektoskop 10. Die Schnittebene VI-VI der Figur 6 ist orthogonal zu der Schnittebene V-V der Figur 5. Die Position der Schnittebene VI-VI der Figur 6 ist in Figur 5 angedeutet. Die Position der Schnittebene V-V der Figur 5 ist in Figur 6 angedeutet.

Die Merkmale der anhand der Figuren 1 bis 3 und der anhand der Figuren 4 bis 6 dargestellten Resektoskope sind miteinander kombinierbar. Insbesondere kann auch bei dem anhand der Figuren 1 bis 3 dargestellten Resektoskop ein Führungsrohr 14, wie es anhand der Figuren 4 bis 6 beschrieben ist, vorgesehen sein. Dabei liegt der O-Ring 85 in der ringförmigen Nut 84 in dem Endoskopschaftkanal 74 insbesondere an der äußeren Oberfläche des Führungsrohrs 14 an.

Ferner können bei dem anhand der Figuren 1 bis 3 dargestellten Resektoskop ähnlich wie bei dem anhand der Figuren 4 bis 6 dargestellten Resektoskop die ringförmige Nut 84 in dem Endoskopschaftkanal 74 und der O-Ring 85 in der ringförmigen Nut 84 und/oder die ringförmige Nut 86 in dem Sondenschaftkanal 76 und der O-Ring 87 in der ringförmigen Nut 86 entfallen.

Figur 7 zeigt eine schematische Darstellung eines Längsschnitts durch einen mittleren Bereich eines weiteren Resektoskops 10, das in einigen Merkmalen, Eigenschaften und Funktionen den anhand der Figuren 1 bis 6 dargestellten Resektoskopen ähnelt, insbesondere auch hinsichtlich des in Figur 7 nicht dargestellten distalen Endes des Resektoskops 10. Die Schnittebene der Figur 7 entspricht den Schnittebenen der Figuren 1, 2, 4 und 5. Die in Figur 7 gezeigte Konfiguration entspricht den in den Figuren 1 und 4 gezeigten Konfigurationen. Nachfolgend sind insbesondere Merkmale, Eigenschaften und Funktionen des in Figur 7 gezeigten Resektoskops beschrieben, in denen diese sich von den anhand der Figuren 1 bis 6 dargestellten Resektoskopen unterscheiden.

Das in Figur 7 gezeigte Resektoskop 10 unterscheidet sich von den anhand der Figuren 1 bis 6 dargestellten Resektoskopen insbesondere dadurch, dass die Dichteinrichtung 70 durch eine Renkverbindung mit dem distalen Ende 32 des Arbeitselements 30 verbunden ist. An dem distalen Ende 32 des Arbeitselements 30 sind anstelle eines ringförmigen Kragens 37 oder - abweichend von dem hier dargestellten Beispiel - zusätzlich zu diesem zwei oder mehr Knaggen 88 vorgesehen. An der Dichteinrichtung 70 ist eine entsprechende Anzahl korrespondierender und jeweils L-förmiger Nuten 89 vorgesehen. Jede L-förmige Nut 89 weist einen axialen Abschnitt parallel zu den Längsachsen des Resektoskopschafts 20, des Endoskopschafts 42 und des zylindrischen Abschnitts 66 des Sondenschafts und einen sich in umfänglicher Richtung erstreckenden Abschnitt auf. Bei der in Figur 7 gezeigten Konfiguration greifen die Knaggen 88 an dem distalen Ende 32 des Arbeitselements 30 in die sich in umfänglicher Richtung erstreckenden Abschnitte der L-förmigen Nuten 89 an der Dichteinrichtung 70 ein und bilden so eine formschlüssige Verbindung zwischen dem distalen Ende 32 des Arbeitselements 30 und der Dichteinrichtung 70.

Figur 8 zeigt eine weitere schematische Darstellung eines Schnitts durch das anhand der Figur 7 dargestellte Resektoskop 10. Die Schnittebene VIII-VIII der Figur 8 entspricht der Schnittebene der Figur 7 und den Schnittebenen der Figuren 1, 2, 4 und 5. Die in Figur 8 gezeigte Konfiguration entspricht den in den Figuren 2 und 5 dargestellten Konfigurationen.

Bei der in Figur 8 gezeigten Anordnung der Dichteinrichtung 70 räumlich beabstandet von dem distalen Ende 32 des Arbeitselements 30 sind die L-förmigen Nuten 89 an der Dichteinrichtung 70 erkennbar. Die in Figur 8 gezeigte Konfiguration ist ausgehend von der in Figur 7 gezeigten Konfiguration erreichbar, indem zunächst die Dichteinrichtung 70 relativ zu dem distalen Ende 32 des Arbeitselements 30 um einen vorbestimmten Winkel gedreht wird, um die Knaggen 88 an dem distalen Ende 32 des Arbeitselements 30 bis in den Übergangsbereich zwischen den umfänglichen Abschnitten und den axialen Abschnitten der L-förmigen Nuten 89 an der Dichteinrichtung 70 zu bewegen. Danach kann die Dichteinrichtung 70 in axialer Richtung von dem distalen Ende 32 des Arbeitselements 30 getrennt werden.

Figur 9 zeigt eine weitere schematische Darstellung eines Schnitts durch das Resektoskop 10 aus den Figuren 7 und 8. Die Schnittebene IX-IX der Figur 8 ist orthogonal zu der Schnittebene VIII-VIII der Figur 8 und parallel zu den Schnittebenen der Figuren 3 und 6. Die Position der Schnittebene VIII-VIII der Figur 8 ist in Figur 9 angedeutet. Die Position der Schnittebene IX-IX der Figur 9 ist in Figur 8 angedeutet.

Im Gegensatz zu der Schnittebene III-III der Figur 3 und der Schnittebene VI-VI der Figur 6 schneidet die Schnittebene IX-IX der Figur 9 nicht die Dichteinrichtung 70, sondern das distale Ende 32 des Arbeitselements 30.

In Figur 9 ist der bereits erwähnte Schlitz 35 in dem Arbeitselement 30 sichtbar, der ein seitliches Einlegen des zylindrischen Abschnitts 66 des Sondenschafts 60 in die zweite Öffnung 36 in dem distalen Ende 32 des Arbeitselements 30 ermöglicht. Der Schlitz 35 reicht von einem Umfang des distalen Endes 32 des Arbeitselements 30 bis zu der als Durchgangskanal ausgebildeten zweiten Öffnung 36. Zum Einlegen des zylindrischen Abschnitts 66 des Sondenschafts 60 in die zweite Öffnung 36 oder zum Entnehmen des zylindrischen Abschnitts 66 des Sondenschafts 60 aus der zweiten Öffnung 36 mit einer Bewegung in Richtung parallel zu der Schnittebene IX-IX der Figur 9 kann der Kupplungsring 38 in eine Position gedreht werden, in der ein Schlitz in dem Kupplungsring 38 in Deckung ist mit dem Schlitz 35 in dem distalen Ende 32 des Arbeitselements 30.

In Figur 9 ist ferner erkennbar, dass bei dem anhand der Figuren 7 bis 9 dargestellten Resektoskop 10 sowohl die erste Öffnung 34 als auch die zweite Öffnung 36 in dem distalen Ende 32 des Arbeitselements 30 jeweils etwas größer als die Querschnitte des Endoskopschafts 42 bzw. des zylindrischen Abschnitts 66 des Sondenschafts 60 ausgebildet sind, so dass die Dichteinrichtung 70 zusammen mit dem Endoskopschaft 42 und dem zylindrischen Abschnitt 66 des Sondenschafts 60, die in dem Endoskopschaftkanal 74 bzw. dem Sondenschaftkanal 76 der Dichteinrichtung 70 (vgl. Figuren 7, 8) spielarm geführt sind, relativ zu dem distalen Ende 32 des Arbeitselements 30 um einen vorbestimmten Winkel rotiert werden können, um die anhand der Figuren 7 und 8 beschriebene Renkverbindung zwischen der Dichteinrichtung 70 und dem distalen Ende 32 des Arbeitselements 30 zu bilden oder zu lösen. Bei dieser Rotationsbewegung erreichbare Positionen der Querschnitte des Endoskopschafts 42 und des Sondenschafts 66 sind in Figur 9 durch gestrichelte Linien angedeutet.

Figur 10 zeigt eine schematische Darstellung eines Längsschnitts durch ein weiteres Resektoskop 10, das in einigen Merkmalen, Eigenschaften und Funktionen den anhand der Figuren 1 bis 9 dargestellten Resektoskopen ähnelt. Die Schnittebene der Figur 10 entspricht den Schnittebenen der Figuren 1, 2, 4, 5, 7, 8. Die in Figur 10 gezeigte Konfiguration entspricht den in den Figuren 1, 4, 7 gezeigten Konfigurationen. Nachfolgend sind insbesondere Merkmale, Eigenschaften und Funktionen des in Figur 10 gezeigten Resektoskops beschrieben, in denen diese sich von den anhand der Figuren 1 bis 9 dargestellten Resektoskopen unterscheiden.

Das in Figur 10 gezeigte Resektoskop unterscheidet sich von den anhand der Figuren 1 bis 9 dargestellten Resektoskopen insbesondere dadurch, dass an dem distalen Ende 32 des Arbeitselements 30 Ausnehmungen 93 mit Stufen 94 vorgesehen sind, in die biegeelastische Holme 97 an der Dichteinrichtung 70 eingreifen. An den freien Enden der biegeelastischen Holme 97 der Dichteinrichtung 70 sind Rastnasen 98 angeordnet, die bei der in Figur 10 gezeigten Konfiguration die Stufen 94 in den Ausnehmungen 93 hintergreifen und so die Dichteinrichtung 70 an dem distalen Ende 32 des Arbeitselements 30 formschlüssig halten.

Figur 11 zeigt eine weitere schematische Darstellung eines Schnitts durch das anhand der Figur 10 dargestellte Resektoskop 10. Die Schnittebene XI-XI der Figur 11 entspricht der Schnittebene der Figur 10 und den Schnittebenen der Figuren 1, 2, 4, 5, 7, 8. Die in Figur 11 gezeigte Konfiguration entspricht den anhand der Figuren 2, 5, 8 dargestellten Konfigurationen.

Ausgehend von der in Figur 10 gezeigten Konfiguration erfordert die Bewegung der Dichteinrichtung 70 in die in Figur 11 gezeigte Position relativ zu dem distalen Ende 32 des Arbeitselements 30 zunächst eine Auslenkung der Rastnasen 98 und damit eine elastische Verformung der biegeelastischen Holme 97 gegen deren elastische Rückstellkräfte. Ein manuelles Abziehen der Dichteinrichtung 70 von dem distalen Ende 32 des Arbeitselements 30 erfordert deshalb eine vorbestimmte Kraft, die von der genauen Geometrie der Stufen 94, der Rastnasen 98 und der biegeelastischen Holme 97 und den elastischen Eigenschaften des die biegeelastischen Holme 97 bildenden Materials abhängt. Die vorbestimmte Kraft ist insbesondere so eingestellt, dass einerseits beim Trennen des proximalen Endes 23 des Resektoskopschafts 20 von dem distalen Ende 32 des Arbeitselements 30 mit ausreichender Sicherheit verhindert wird, dass die Dichteinrichtung 70 an dem proximalen Ende 23 des Resektoskopschafts 20 verbleibt, und dass andererseits die Dichteinrichtung 70 anschließend ausreichend leicht von dem distalen Ende 32 des Arbeitselements 30 getrennt werden kann.

Figur 12 zeigt eine schematische Darstellung eines weiteren Schnitts durch das anhand der Figuren 10 und 11 dargestellte Resektoskop 10. Die Schnittebene XII-XII der Figur 12 ist orthogonal zu der Schnittebene XI-XI der Figur 11 und entspricht der Schnittebene IX-IX der Figur 9. Die Position der Schnittebene XII-XII der Figur 12 ist in Figur 11 angedeutet. Die Position der Schnittebene XI-XI der Figur 11 ist in Figur 12 angedeutet.

Die Ausnehmungen 93 an dem distalen Ende 32 des Arbeitselements 30 sind bei dem dargestellten Beispiel jeweils taschenförmig mit rechteckigen Querschnitten.

### Bezugszeichen

- 10: Resektoskop
- 14: Führungsrohr
- 20: Resektoskopschaft des Resektoskops 10
- 21: distales Ende des Resektoskopschafts 10
- 22: aus einem elektrisch isolierenden Material gebildeter Abschnitt des Resektoskopschafts 10 an dessen distalem Ende 21
- 23: proximales Ende des Resektoskopschafts 20
- 24: Führungsrohr in dem Resektoskopschaft 20 zur Aufnahme des Schafts 40 des Endoskops 40
- 27: konische Dichtfläche an dem proximalen Ende 23 des Resektoskopschafts 20
- 29: Knagge an dem proximalen Ende 23 des Resektoskopschafts 20
- 30: Arbeitselement des Resektoskops 10
- 32: distales Ende des Arbeitselements 30
- 34: erste Öffnung in dem distalen Ende 32 des Arbeitselements 30 zur Aufnahme des Endoskopschafts 42 des Endoskops 40
- 35: Schlitz in dem distalen Ende 32 des Arbeitselements 30, vom Umfang des Arbeitselements 30 bis zu der ersten Öffnung 34 reichend
- 36: zweite Öffnung in dem distalen Ende 32 des Arbeitselements 30 zur Aufnahme des zylindrischen Abschnitts 66 des Sondenschafts 60
- 37: Kragen an dem distalen Ende 32 des Arbeitselements 30, zur Zentrierung der Dichteinrichtung 70
- 38: Kupplungsring an dem distalen Ende 32 des Arbeitselements 30
- 39: Knagge an dem Kupplungsring 38
- 40: Endoskop des Resektoskops 10
- 41: distales Ende des Endoskops 10
- 42: Endoskopschaft des Endoskops 40
- 50: Sondeneinrichtung des Resektoskops 10
- 51: distales Ende der Sondeneinrichtung 50
- 52: Elektrode als Wirkeinrichtung der Sondeneinrichtung 50
- 54: Führungseinrichtung der Sondeneinrichtung 50 zur Führung an dem Endoskopschaft 42
- 60: Sondenschaft der Sondeneinrichtung 50
- 66: zylindrischer Abschnitt des Sondenschafts 60
- 70: Dichteinrichtung der Sondeneinrichtung 50
- 72: konische Dichtfläche an der Dichteinrichtung 70
- 73: umlaufende Stufe an der Dichteinrichtung 70
- 74: Endoskopschaftkanal der Dichteinrichtung 70, zur Aufnahme des Endoskoschafts 42 des Endoskops 40
- 76: Sondenschaftkanal der Dichteinrichtung 70, zur Aufnahme des Sondenschafts 60
- 84: ringförmige Nut in dem Endoskopschaftkanal 74
- 85: O-Ring in der ringförmigen Nut 84 in dem Endoskopschaftkanal 74
- 86: ringförmige Nut in dem Sondenschaftkanal 76
- 87: O-Ring in der ringförmigen Nut 86 in dem Sondenschaftkanal 76
- 88: Knagge an dem distalen Ende 32 des Arbeitselements 30
- 89: L-förmige Nut an der Dichteinrichtung 70, zur Aufnahme der Knagge 88 an dem distalen Ende 32 des Arbeitskanals 30
- 93: Ausnehmung an dem distalen Ende 32 des Arbeitselements 30
- 94: Stufe in der Ausnehmung 93
- 97: biegeelastischer Holm an der Dichteinrichtung 70
- 98: Rastnase an dem biegeelastischen Holm 97

## Patentansprüche

1. **Sondeneinrichtung** (50) für ein Resektoskop (10) oder ein anderes mikroinvasives medizinisches Instrument zur Bearbeitung oder Manipulation von Gewebe, mit:
einem **Sondenschaft** (60) mit einem zylindrischen Abschnitt (66) zur Anordnung in einem Instrumentenschaft (20) und einem distalen Ende zur Anordnung nahe einem distalen Ende des Instrumentenschafts (20);
einer **Wirkeinrichtung** (52) an dem distalen Ende des Sondenschafts (60);
einer **Dichteinrichtung** (70) mit einem **Sondenschaftkanal** (76), in dem der zylindrische Abschnitt (66) des Sondenschafts (60) angeordnet ist, wobei der Sondenschaft (60) relativ zu der Dichteinrichtung (70) parallel zu der Längsachse des Sondenschafts (60) und parallel zu der Längsachse des Sondenschaftkanals (76) verschiebbar ist,
**dadurch gekennzeichnet, dass** die Dichteinrichtung (70) aus **Metall** oder **Keramik** oder aus einem anderen nicht elastischen Material gebildet und **dauerhaft** mit dem Sondenschaft (60) **verbunden** ist,
wobei das nicht elastische Material einen Elastizitätsmodul größer als 10 GPa aufweist.

2. Sondeneinrichtung (50) nach einem der vorangehenden Ansprüche, bei der
die Dichteinrichtung (70) eine **konische Dichtfläche** (72) aufweist, die zur dichtenden Anlage an einer korrespondierenden konischen Dichtfläche (27) an einem Instrumentenschaft (20) vorgesehen und ausgebildet ist.

3. Sondeneinrichtung (50) nach einem der vorangehenden Ansprüche, bei der
der Sondenschaftkanal (76) eine **ringförmige Erweiterung** (86) aufweist, in der ein **Dichtelement** (87) angeordnet ist.

4. Sondeneinrichtung (50) nach einem der vorangehenden Ansprüche, bei der
die Dichteinrichtung (70) eine **Kupplungseinrichtung** (89; 97, 98) zur lösbaren mechanischen Verbindung der Dichteinrichtung (70) mit dem distalen Ende (32) eines Arbeitselements (30) eines Resektoskops (10) oder eines anderen mikroinvasiven Instruments aufweist.

5. Sondeneinrichtung (50) nach dem vorangehenden Anspruch, bei der
die Dichteinrichtung (70) eine elastische **Rasteinrichtung** (97, 98) zur Ausbildung einer Rastverbindung mit dem distalen Ende (32) des Arbeitselements (30) des Resektoskops (10) oder des anderen mikroinvasiven Instruments umfasst.

6. Sondeneinrichtung (50) nach Anspruch 4, bei der
die Kupplungseinrichtung eine Nut oder eine andere **Ausnehmung** zur Aufnahme eines elastischen Rastelements an dem distalen Ende (32) des Arbeitselements (30) des Resektoskops (10) oder des anderen mikroinvasiven Instruments aufweist.

7. Sondeneinrichtung (50) nach Anspruch 4, bei der
die Dichteinrichtung (70) eine **Knagge** oder eine L-förmige Nut (89) zur Ausbildung einer Renkverbindung mit dem distalen Ende (32) des Arbeitselements (30) des Resektoskops (10) oder des anderen mikroinvasiven Instruments umfasst.

8. **Mikroinvasives medizinisches Instrument zur Bearbeitung oder Manipulation von Gewebe** (10) mit:
einer Sondeneinrichtung (50) gemäß einem der vorangehenden Ansprüche;
einem **Instrumentenschaft** (20) mit einem distalen Ende und einem proximalen Ende (23), zur Aufnahme des Sondenschafts (60) der Sondeneinrichtung (50);
einem **Arbeitselement** (30) zur lösbaren Verbindung mit dem proximalen Ende (23) des Instrumentenschafts (20) und zur lösbaren Verbindung mit einem proximalen Ende eines Endoskops (40) und zur lösbaren Verbindung mit einem proximalen Ende des Sondenschafts (60) und zur manuellen Bewegung des Sondenschafts (60) relativ zu dem Instrumentenschaft (20),
wobei zwischen dem distalen Ende (32) des Arbeitselements (30) und dem proximalen Ende (23) des Instrumentenschafts (20) ein Raum zur Aufnahme der Dichteinrichtung (70) der Sondeneinrichtung (50) vorgesehen ist.

9. Mikroinvasives Instrument (10) nach dem vorangehenden Anspruch, bei dem
an dem proximalen Ende (23) des Instrumentenschafts (20) eine konische Dichtfläche (27) vorgesehen ist, die zumindest teilweise zu einer konischen Dichtfläche (72) einer Dichteinrichtung (70) einer Sondeneinrichtung (50) nach einem der Ansprüche 1 bis 7 korrespondiert.

10. Mikroinvasives Instrument (10) nach dem vorangehenden Anspruch, bei dem
an dem distalen Ende (32) des Arbeitselements (30) eine **Öffnung** (34, 36) für den Sondenschaft (60) oder für den Endoskopschaft (42) vorgesehen ist,
die Öffnung (34, 36) für den Sondenschaft (60) oder für den Endoskopschaft (42) einen Querschnitt aufweist, der eine **Bewegung** des Sondenschafts (60) oder des Endoskopschafts (42) **in der Öffnung** (34, 36) bei einem Rotieren der Dichteinrichtung (70) relativ zu dem distalen Ende (32) des Arbeitselements (30) ermöglicht.

11. Mikroinvasives Instrument nach einem der vorangehenden Ansprüche, bei dem das mikroinvasive Instrument ein **Resektoskop** (10) ist.

## Claims

1. **A probe device** (50) for a resectoscope (10) or other microinvasive medical instrument for processing or manipulating tissue, comprising:
a **probe shaft** (60) having a cylindrical section (66) for arrangement in an instrument shaft (20) and a distal end for arrangement near a distal end of the instrument shaft (20);
an **effecting device** (52) at the distal end of the probe shaft (60);
a **sealing device** (70) with a **probe shaft channel** (76), in which the cylindrical section (66) of the probe shaft (60) is arranged, wherein the probe shaft (60) is displaceable relative to the sealing device (70) parallel to the longitudinal axis of the probe shaft (60) and parallel to the longitudinal axis of the probe shaft channel (76),
**characterised in that**
the sealing device (70) is made of **metal or ceramic** or another non-elastic material and is **permanently connected** to the probe shaft (60),
wherein the non-elastic material has a modulus of elasticity greater than 10 GPa.

2. The probe device (50) according to one of the preceding claims, in which
the sealing device (70) has a **conical sealing surface** (72) which is provided and designed for sealing contact with a corresponding conical sealing surface (27) on an instrument shaft (20).

3. The probe device (50) according to one of the preceding claims, in which
the probe shaft channel (76) has an **annular extension** (86) in which a **sealing element** (87) is arranged.

4. The probe device (50) according to one of the preceding claims, in which
the sealing device (70) has a **coupling device** (89; 97, 98) for detachable mechanical connection of the sealing device (70) to the distal end (32) of a working element (30) of a resectoscope (10) or another microinvasive instrument.

5. The probe device (50) according to the preceding claim, in which
the sealing means (70) comprises an elastic **latching device** (97, 98) for forming a latched connection with the distal end (32) of the working element (30) of the resectoscope (10) or the other microinvasive instrument.

6. The probe device (50) according to claim 4, in which
the coupling device has a groove or other **recess** for receiving a elastic latching element at the distal end (32) of the working element (30) of the resectoscope (10) or the other microinvasive instrument.

7. The probe device (50) according to claim 4, in which
the sealing device (70) comprises a **catch** or an L-shaped groove (89) for forming a bayonet connection with the distal end (32) of the working element (30) of the resectoscope (10) or the other microinvasive instrument.

8. **A microinvasive medical instrument for processing or manipulating tissue** (10) comprising:
a probe device (50) according to one of the preceding claims;
an **instrument shaft** (20) having a distal end and a proximal end (23), for receiving the probe shaft (60) of the probe device (50);
a **working element** (30) for detachably connecting to the proximal end (23) of the instrument shaft (20) and for detachably connecting to a proximal end of an endoscope (40) and for detachably connecting to a proximal end of the probe shaft (60) and for manually moving the probe shaft (60) relative to the instrument shaft (20),
wherein a space for receiving the sealing device (70) of the probe device (50) is provided between the distal end (32) of the working element (30) and the proximal end (23) of the instrument shaft (20).

9. The microinvasive instrument (10) according to the preceding claim, in which
at the proximal end (23) of the instrument shaft (20) is provided a conical sealing surface (27), which at least partially corresponds to a conical sealing surface (72) of a sealing device (70) of a probe device (50) according to one of claims 1 to 7.

10. The microinvasive instrument (10) according to the preceding claim, in which
an **opening** (34, 36) for the probe shaft (60) or for the endoscope shaft (42) is provided at the distal end (32) of the working element (30),
the opening (34, 36) for the probe shaft (60) or for the endoscope shaft (42) has a cross-section which allows a **movement** of the probe shaft (60) or of the endoscope shaft (42) **in the opening** (34, 36) when the sealing device (70) is rotated relative to the distal end (32) of the working element (30).

11. The microinvasive instrument according to one of the preceding claims, in which the microinvasive instrument is a **resectoscope** (10).

## Revendications

1. **Dispositif de sonde** (50) pour un résectoscope (10) ou un autre instrument médical micro-invasif pour le traitement ou la manipulation de tissus, comportant :
une **tige de sonde** (60) comportant une section cylindrique (66) pour l'agencement dans une tige d'instrument (20) et une extrémité distale pour l'agencement à proximité d'une extrémité distale de la tige d'instrument (20) ;
un **dispositif d'action** (52) à l'extrémité distale de la tige de sonde (60) ;
un **dispositif d'étanchéité** (70) comportant un **canal de tige de sonde** (76) dans lequel la section cylindrique (66) de la tige de sonde (60) est agencée, dans lequel la tige de sonde (60) peut être déplacée par rapport au dispositif d'étanchéité (70) parallèlement à l'axe longitudinal de la tige de sonde (60) et parallèlement à l'axe longitudinal du canal de tige de sonde (76),
**caractérisé en ce que**
le dispositif d'étanchéité (70) est formé de **métal** ou de **céramique** ou d'un autre matériau non élastique et est **relié durablement** avec la tige de sonde (60),
dans lequel le matériau non élastique présente un module d'élasticité supérieur à 10 GPa.

2. Dispositif de sonde (50) selon l'une des revendications précédentes, dans lequel
le dispositif d'étanchéité (70) présente une **surface d'étanchéité conique** (72), qui est prévue et réalisée pour s'appliquer de manière étanche contre une surface d'étanchéité conique (27) correspondante sur une tige d'instrument (20).

3. Dispositif de sonde (50) selon l'une des revendications précédentes, dans lequel
le canal de tige de sonde (76) présente un **élargissement annulaire** (86), dans lequel est agencé un **élément d'étanchéité** (87).

4. Dispositif de sonde (50) selon l'une des revendications précédentes, dans lequel
le dispositif d'étanchéité (70) comprend un **dispositif d'accouplement** (89 ; 97, 98) pour relier mécaniquement de manière amovible le dispositif d'étanchéité (70) à l'extrémité distale (32) d'un élément de travail (30) d'un résectoscope (10) ou d'un autre instrument micro-invasif.

5. Dispositif de sonde (50) selon la revendication précédente, dans lequel
le dispositif d'étanchéité (70) comprend un **dispositif d'encliquetage** (97, 98) élastique pour la formation d'une liaison par encliquetage avec l'extrémité distale (32) de l'élément de travail (30) du résectoscope (10) ou de l'autre instrument micro-invasif.

6. Dispositif de sonde (50) selon la revendication 4, dans lequel
le dispositif d'accouplement présente une rainure ou un autre **évidement** pour la réception d'un élément d'encliquetage élastique à l'extrémité distale (32) de l'élément de travail (30) du résectoscope (10) ou de l'autre instrument micro-invasif.

7. Dispositif de sonde (50) selon la revendication 4, dans lequel
le dispositif d'étanchéité (70) comprend un **taquet** ou une rainure en forme de L (89) pour la formation d'une liaison à rotule avec l'extrémité distale (32) de l'élément de travail (30) du résectoscope (10) ou de l'autre instrument micro-invasif.

8. **Instrument médical micro-invasif pour le traitement ou la manipulation de tissus** (10) comportant :
un dispositif de sonde (50) selon l'une des revendications précédentes ;
une **tige d'instrument** (20) comportant une extrémité distale et une extrémité proximale (23), pour la réception de la tige de sonde (60) du dispositif de sonde (50) ;
un **élément de travail** (30) pour une liaison amovible à l'extrémité proximale (23) de la tige d'instrument (20) et pour une liaison amovible à une extrémité proximale d'un endoscope (40) et pour une liaison amovible à une extrémité proximale de la tige de sonde (60) et pour le déplacement manuel de la tige de sonde (60) par rapport à la tige d'instrument (20),
dans lequel, entre l'extrémité distale (32) de l'élément de travail (30) et l'extrémité proximale (23) de la tige d'instrument (20), un espace est prévu pour la réception du dispositif d'étanchéité (70) du dispositif de sonde (50).

9. Instrument micro-invasif (10) selon la revendication précédente, dans lequel
une surface d'étanchéité conique (27) est prévue à l'extrémité proximale (23) de la tige d'instrument (20), qui correspond au moins partiellement à une surface d'étanchéité conique (72) d'un dispositif d'étanchéité (70) d'un dispositif de sonde (50) selon l'une des revendications 1 à 7.

10. Instrument micro-invasif (10) selon la revendication précédente, dans lequel
une **ouverture** (34, 36) pour la tige de sonde (60) ou pour la tige d'endoscope (42) est prévue à l'extrémité distale (32) de l'élément de travail (30),
l'ouverture (34, 36) pour la tige de sonde (60) ou pour la tige d'endoscope (42) présente une section transversale qui permet un **déplacement** de la tige de sonde (60) ou de la tige d'endoscope (42) **dans l'ouverture** (34, 36) lors de la rotation du dispositif d'étanchéité (70) par rapport à l'extrémité distale (32) de l'élément de travail (30).

11. Instrument micro-invasif selon l'une des revendications précédentes, dans lequel l'instrument micro-invasif est un **résectoscope** (10).
